# EUROPEAN PATENT SPECIFICATION

(11) **EP 1 078 005 B1**
(45) Date of publication and mention of the grant of the patent: **21.04.2010**
(21) Application number: 99923260.6
(22) Date of filing: 21.05.1999
(51) Int. Cl.: C07K 16/18, A61K 39/395

(54) **METHODS FOR AMYLOID REMOVAL USING ANTI-AMYLOID ANTIBODIES**
METHODEN ZUR AMYLOIDENTFERNUNG MIT ANTI-AMYLOID-ANTIKÖRPER
PROCEDE D'ELIMINATION D'AMYLOIDE A L'AIDE D'ANTICORPS ANTI-AMYLOIDE

(30) Priority: 21.05.1998 US 86198 P
(43) Date of publication of application: 28.02.2001
(73) Proprietor: UNIVERSITY OF TENNESSEE RESEARCH FOUNDATION, Knoxville, TN 37996 (US)
(72) Inventor: SOLOMON, Alan, Knoxville, TN 37914 (US); HRNCIC, Rudi, Knoxville, TN 37923 (US); WALL, Jonathan, S., Knoxville, TN 37919 (US)
(74) Representative: Walker, Ross Thomson
(86) International application number: PCT/US1999/011200
(87) International publication number: WO 1999/060024

(56) References cited:
- WO-A-99/27944
- WO-A1-89/01343
- WO-A1-96/25435
- Bioconjugate Chem., Volume 5, 1994, Ulrich Bickel et al, "Development and in Vitro Characterization of a Cationized Monoclonal Antibody against BetaA4 Protein: A Potential Probe for Alzheimer's Disease1"
- Journal of Neuropatholy and Experimental Neurology, Volume 53, No. 4, July 1994, Lary C. Walker et al, "Labeling of Cerebral Amyloid in Vivo with a Monoclonal Antibody"
- SOLOMON B. ET AL: PROC.NATL.ACAD.SCI., vol. 94, no. 8, 15 April 1997 (1997-04-15), pages 4109-4112, XP002911322
- BELLOTTI V. ET AL: "Use of an Anti-Idiotypic Monoclonal Antibodies in Studying Amyloidogenic Light Chains in Cells, Urine and Fibrils: Pathophysiology and Clinical Implications" SCAND.J.IMMUNOL., vol. 36, 1992, pages 607-615, XP009086264
- YU Z. ET AL: INT. J. CANCER, vol. 56, 1994, pages 244-248, XP000946044
- KAMETANI F. ET AL: "A monoclonal antibody Hy20-54-16-3L to lambda light chain of human immunoglobulin reacts with amyloid in Alzheimer's disease brain" NEUROSCIENCE LETTERS, vol. 117, 1990, pages 62-67, XP009013620

## Description

### Technical Field

The present invention generally relates to compositions for use in treating amyloid-related diseases. Specifically, the present invention provides therapeutic antibody-related uses to effect the removal of amyloid fibrils by a patient's own immunophagocytic system.

### Background of the Invention

Amyloidosis refers to the pathological deposition of proteins in the form of congophilic, green birefringent fibrils, when congo red-stained, either dispersed or in the form of localized amyloidomas. Such deposits are symptomatic of several diseases, for example Alzheimer's Disease, inflammation-associated amyloid, type II diabetes, bovine spongiform encephalopathy (BSE), Creutzfeld-Jakob disease (CJD), scrapie and primary amyloidosis.

Amyloidoses are generally categorized into three groups: major systemic amyloidoses, major localized amyloidoses, and miscellaneous amyloidoses. Major systemic amyloidoses include: chronic inflammatory conditions (*e.g*., tuberculosis, osteomyelitis, etc.); non-infectious conditions such as juvenile rheumatoid arthritis, ankylosing spondylitis and Crohn's disease, *etc*.; familial Mediterranean Fever, plasma cell dyscrasia (primary amyloidosis) and various familial polyneuropathies and cardiomyopathies. Major localized amyloidoses include: chronic dialysis usually for greater than 8 years, Alzheimer's disease, Down syndrome, Hereditary cerebral hemorrhage (Dutch), and non-traumatic cerebral hemorrhage of the elderly. Miscellaneous amyloidoses include: familial polyneuropathy (Iowa), familial amyloidosis (Finnish), hereditary cerebral hemorrhage (Icelandic), CJD, Medullary carcinoma of the thyroid, atrial amyloid, and diabetes mellitus (insulinomas). Other amyloidoses include those referenced in Louis W. Heck, "The Amyloid Diseases" in Cecil's Textbook of Medicine 1504-6 (W.B. Saunders & Co., Philadelphia, PA; 1996).

Transmissible spongiform encephalopathies which cause CJD and Gerstmann-Strässler-Scheinker (GSS) disease are described by B. Chesebro et al., "Transmissible Spongiform Encephalopathies: A Brief Introduction" in FIELD'S VIROLOGY 2845-49 (3rd Edition; Raven Publishers, Philadelphia, PA; 1996) and in D.C. Gajdusek, "Infectious amyloids: Subacute Spongiform Encephalopathies as Transmissible Cerebral Amyloidoses," 2851-2900 in FIELDS VIROLOGY (1996). Many of these diseases are likely mediated by prions, an infectious protein. See S.B. Prusiner, "Prions" in FIELDS VIROLOGY 2901-50 (1996) and the references contained therein. The inherited forms of amyloidoses as described on Online Mendelian Inheritance in Man (OMIM) "www.ncbi.nlm.nih.gov/htbin-post/Omim/dispmim?" Each of the above is incorporated herein by reference.

Very rarely do patients with clinically proven amyloidosis spontaneously achieve complete remission, perhaps because the amyloid fibrils themselves are non-immunogenic. Various therapies for amyloidosis have been investigated, such as high-dose chemotherapy, steroids, iodinated doxorubicin, and stem cell replacement therapy. However, in only one type of amyloid disease, Familial-Mediterranean amyloidosis, has drug treatment (with colchicine) been shown to be effective.

The use of monoclonal antibodies (mAbs) to induce or modulate the immunological removal of an otherwise unrecognized entity is known. mAbs have been successfully used in treating non-Hodgkins lymphoma and breast cancer, for example.

Previously, a variety of studies have characterized antibodies that bind to amyloid proteins or amyloid fibrils. See, for example. U.S. Patents Nos. 5,714,471; 5,693,478; 5,688,651; 5,652,092; 5,593,846; 5,536,640; 5,385,915; 5,348,963; 5,270,165; 5,262,332; 5,262,303; 5,164,295 and 4,782,014. In addition, several publications have suggested that anti-amyloid antibodies might be useful for studying the progression of beta-amyloidosis and for various therapeutic options. See, for example, Bellottii et al., Scand. J. Immunol. (1992) 36(4):607-615; Bellotti et al, Ren. Fail. (1993) 15(3):365-371; Walker et al J. Neuropathol. Exp. Neurol. (1994) 53(4):377-383; and Bickel et al., Bioconjug. Chem. (1994) 5(2):119-125. However, no therapeutic antibody has been demonstrated to halt or reverse the deposition of amyloid fibrils in a patient. Thus, a need exists for a method for treating amyloidoses using antibody formulations containing antibodies that bind to amyloid fibrils.

WO 96/25435 disclose *in vitro* methods of labeling amyloid plaques present in the brain of a human patient, using immunohistochemical techniques involving monoclonal antibodies that bind beta-A4 peptide.

"Bioconjugate Chem.: volume 5, pages 119-125, (1994)" discloses *in vitro* binding of monoclonal antibodies to isolated brain capillaries.

According to the present invention there is provided the use of:
a) a therapeutically effective dose of at least one antibody or immunoglobulin polypeptide or a fragment thereof, wherein the antibody or immunoglobulin polypeptide or fragment thereof binds to human IgLC amyloid fibril; and
b) a pharmaceutically acceptable carrier; for the manufacture of a medicament for the treatment of an amyloid deposition disease in a human patient.

Also in accordance with the present invention there is provided a pharmaceutical composition comprising a therapeutically effective dose of at least one antibody or immunoglobulin polypeptide or a fragment thereof, wherein the antibody or immunoglobulin polypeptide or fragment thereof binds to human IgLC amyloid fibril and is effective to enhance the cellular immune response of a patient to remove amyloid deposits in the patient.

### Brief Description of the Drawings

**Figures 1A and 1B.** Figures 1A and 1B are reproduced photographs of a Balb/c mouse just after an injection of amyloid is made (1A) and 14 days after the injection (1B). The injection site was shaved to better illustrate the "hump" caused by the injection of the amyloid material.
**Figures 2A-2B.** Figures 2A and 2B are reproduced photographs of human neutrophils (multi-lobed nuclei) adhering to human amyloid opsonized *in vitro.*
**Figures 3A-3D.** Figures 3A-3D are reproduced photographs of immunohistochemically stained amyloid-laden tissue samples (20X magnification). Figure 3A is a tissue sample from a patient with κ1 amyloidosis stained with Congo red; the amyloid deposits, viewed under polarized light, appear as blue-green particles. Figure 3B is a tissue sample stained with alkaline phosphatase after labeling with anti-κI (57-18-H12) mAb. Figure 3C is a tissue sample stained as in Figure 3B, but with anti-κIV (11-1F4) mAb. Figure 3D is a tissue sample stained as in Figure 3B, but with anti-λVIII (31-8c7) mAb.
**Figure 4.** Figure 4 is a reproduced photograph showing a fluoresceinated (FITC) κ4 mAb bound to human amyloid implanted into a Balb/c mouse. The mAb was injected into the thigh of the mouse. The amyloidoma was excised 72 hours post injection and viewed using an epifluorescence microscope (20X magnification).

### Modes of Carrying Out the Invention

### General Description

The present invention utilizes immunoglobulin polypeptides to modulate and to enhance the degradation and removal of undesired deposits of amyloid fibrils in a host or patient. It is envisioned that the invention will be used, for example, to treat humans suffering from a disease or condition characterized by an undesired deposition of amyloid fibrils. Without intending to be bound by any particular mechanism of action, it is believed that the administration of immunoglobulin peptides used in the present invention opsonize the deposited amyloid fibrils in a patient suffering from amyloidosis, thereby assisting in their removal from the patient by the patients' own immune system. It is believed that the patient's immune system alone is unable to remove the amyloid fibrils in conditions modulated by amyloid fibrils without such a therapeutic intervention, presumably because the amyloid fibrils are themselves relatively non-immunogenic.

To treat a patient with amyloidosis, a therapeutically effective dose of immunoglobulin polypeptide or fragment thereof is administered together with a pharmaceutically suitable carrier or excipient. Upon the binding or adhering of such immunoglobulin polypeptides to undesired deposits of amyloid fibrils, the latter are believed to be opsonized.

Single or multiple administrations of the compositions of the present invention can be carried out in dosages and by administration protocols known to those skilled in the art for the administration of other therapeutic antibody products. These parameters may be selected and/or optimized by the physician treating a particular patient.

Preferably, a therapeutically effective dose of a pharmaceutical formulation of the present invention should deliver a quantity of anti-IgLC amyloid immunoglobulin polypeptide sufficient to substantially inhibit the undesired deposition of amyloid fibrils or to substantially inhibit the rate of any undesired deposition of amyloid fibrils. More preferably, the formulations should reduce the overall burden of deposited amyloid fibrils in a patient. Further, administration of such formulations should begin shortly after diagnosis of amyloidosis and continue until symptoms are substantially abated and for a period thereafter. In well established cases of disease, loading doses followed by maintenance doses may be required.

### Definitions

The terms "peptide," "polypeptide" or "protein" are used interchangeably herein. The term "substantial identity," when referring to polypeptides, indicates that the polypeptide or protein in question is at least about 30% identical to an entire naturally occurring protein or a portion thereof, usually at least about 70% identical, and preferably at least about 95% identical.

As used herein, the terms "isolated," "substantially pure" and "substantially homogenous" are used interchangeably and describe a protein that has been separated from components which naturally accompany it. A substantially purified protein will typically comprise over about 85% to 90% of a protein sample, more usually about 95%, and preferably will be over about 99% pure. Protein purity or homogeneity may be indicated by a number of means well known in the art, such a polyacrylamide gel electrophoresis of a protein sample, followed by visualizing a single polypeptide band on a polyacrylamide gel upon staining. For certain purposes high resolution will be needed and HPLC or a similar means for purification utilized.

Proteins may be purified to substantial homogeneity by standard techniques well known in the art, including selective precipitation with such substances as ammonium sulfate, column chromatography, immunopurification methods, and others. See, for instance, Scopes, Protein Purification: Principles and Practice, Springer-Verlag: New York (1982).

Antibody purification techniques are well known in the art. Harlow et al., Antibodies: A Laboratory Manual, Cold Spring Harbor Laboratory Press, Cold Spring Harbor (1988), 288-318 describes, for example, purification using ammonium sulfate precipitation, caprlic acid, DEAE, hydroxyapatite chromatography, gel filtration chromatography, protein A beads, and immunoaffinity.

Nucleic acids, as used herein, may be DNA or RNA. When referring to nucleic acids, the term "substantial identity" indicates that the sequences of two nucleic acids, or designated portions thereof, when optimally aligned and compared, are identical, with appropriate nucleotide insertions or deletions, in at least about 80% of the nucleotides, usually at least about 90% to 95%, and more preferably at least about 98% to 99.5% of the nucleotides.

Alternatively, substantial nucleic acid sequence identity exists when a nucleic acid segment will hybridize under selective hybridization conditions, to a complement of another nucleic acid strand.

"Substantially complementary" similarly means that one nucleic acid hybridizes selectively to, or is identical to, another nucleic acid. Typically, selective hybridization will occur when there is at least about 55% identity over a stretch of at least 14-25 ) nucleotides, preferably at least about 65% identity, more preferably at least about 75%, and most preferably at least about 90% identity. See M. Kanehisa Nucleic Acids Res. 12:203 (1984).

Stringent hybridization conditions will typically include salt concentrations of less than about 1 M, more usually less than about 500 mM and preferably less than about 200 mM. Temperature conditions will typically be greater than 22° C, typically greater than about 30° C and preferably in excess of about 37° C. As other factors may dramatically affect the stringency of hybridization, including base composition and size of the complementary strands, presence of organic solvents and extent of base mismatching, the combination of parameters is more important than the absolute measure of any one alone.

"Isolated" or "substantially pure," when referring to nucleic acids, refer to those that have been purified away from other cellular components or other contaminants, *e.g.,* other cellular nucleic acids or proteins, by standard techniques, including alkaline/SDS treatment, CsCl banding, column chromatography, and others well known in the art. See, F. Ausubel, et al., ed. Current Protocols in Molecular Biology, Greene Publishing and Wiley-Interscience, New York (1987), incorporated herein by reference.

A nucleic acid is "operably linked" when it is placed into a functional relationship with another nucleic acid sequence. For instance, a promoter or enhancer is operably linked to a coding sequence if it affects the transcription of the sequence. Generally, operably linked means that the nucleic acid sequences being linked are contiguous and, where necessary to join two protein coding regions, contiguous and in reading frame.

Techniques for nucleic acid manipulation, such as subcloning nucleic acid sequences encoding polypeptides into expression vectors, labelling probes, DNA hybridization, and so on are described generally, for example in Sambrook et al., (1989) Molecular Cloning: A Laboratory Manual (2nd ed.), Vols. 1-3, Cold Spring Harbor Laboratory, or Ausubel *et al*., ed. (1987) op. cit., both of which are incorporated herein by reference.

"Expression vectors," "cloning vectors," or "vectors" are often plasmids or other nucleic acid molecules that are able to replicate in a chosen host cell. Expression vectors may replicate autonomously, or they may replicate by being inserted into a genome of the host cell, by methods well known in the art. Vectors that replicate autonomously will have an origin of replication or autonomous replicating sequence ("ARS") that is functional in the chosen host cell(s). Often, it is desirable for a vector to be usable in more than one host cell, *e.g.,* in *E. coli* for cloning and construction, and in a mammalian cell for expression.

Mammalian cell lines are often used as host cells for the expression of polypeptides derived from eukaryotes. Propagation of mammalian cells in culture is per se well known. See, Tissue Culture, Academic Press, Kruse and Patterson, ed. (1973), . Host cell lines may also include such organisms as bacteria (*e.g.*, E. coli or B. subtilis), yeast, filamentous fungi, plant cells, or insect cells, among others.

"Transformation" refers to the introduction of vectors containing the nucleic acids of interest directly into host cells by well-known methods. Transformation methods, which vary depending on the type of host cell, include electroporation; transfection employing calcium chloride, rubidium chloride calcium phosphate, DEAE-dextran, or other substances; microprojectile bombardment; lipofection; infection (where the vector is an infectious agent); and other methods. See generally, Sambrook *et al.,* (1989) op. cit. Reference to cells into which the nucleic acids described above have been introduced is meant to also include the progeny of such cells.

As used herein, "immunoglobulin polypeptide" refers to molecules that are derived from native immunoglobulins (*e.g.*, antibodies) that have specific immunoreactive activity against a particular target, *e.g.,* against amyloid fibrils. Antibodies are typically tetramers of immunoglobulin polypeptides. As used herein, the term "antibody" also refers to a protein consisting of one or more polypeptides substantially encoded by immunoglobulin genes. Immunoglobulin genes include those coding for the light chains, which may be of the kappa or lambda types, and those coding for the heavy chains. Heavy chain types are alpha, gamma, delta, epsilon and mu. The carboxy terminal portions of immunoglobulin heavy and light chains are constant regions, while the amino terminal portions are encoded by the myriad immunoglobulin variable region genes. The variable regions of an immunoglobulin are the portions that provide antigen recognition specificity. In particular, the specificity resides in the complementarity determining regions ("CDRs"), also known as hypervariable regions, of the immunoglobulins.

The immunoglobulins may exist in a variety of fragment forms including, for example, Fv, Fab, F(ab"), F(ab')₂, SvFv and other fragments, as well as single chains (*e.g.*, Huston, et al., Proc. Nat. Acad. Sci. U.S.A., 85:5879-5883 (1988) and Bird, et al., Science 242:423-426 (1988). (See, generally, Hood, et al., "Immunology," Benjamin, N.Y., 2nd ed. (1984), and Hunkapiller and Hood, Nature, 323:15-16 (1986)). Single-chain antibodies, in which genes for a heavy chain and a light chain are combined into a single coding sequence, may also be used. Immunoglobulin polypeptide also encompasses a truncated immunoglobulin chain, for example, a chain containing less constant region domains than in the native polypeptide. Such truncated polypeptides can be produced by standard methods such as introducing a stop codon into the gene sequence 5' of the domain sequences to be deleted. The truncated polypeptides can then be assembled into truncated antibodies. Antibodies as used herein also include bispecific antibodies which can be produced such as by the methods described in the following references: Glennie et al., J. Immunol., 139:2367-2375 (1987); Segal et al., Biologic Therapy of Cancer Therapy of Cancer Updates 2(4):1-12 (1992); and Shalaby et al., J. Exp. Med. 175:217-225 (1992).

"Monoclonal antibodies" may be obtained by various techniques familiar to those skilled in the art. Briefly, spleen cells from an animal immunized with a desired antigen are immortalized, commonly by fusion with a myeloma cell (see, Kohler and Milstein, Eur. J. Immunol, 6:511-519 (1976)). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host.

Monospecific and bispecific immunoglobulins may also be produced by recombinant techniques in prokaryotic or eukaryotic host cells.

"Chimeric" antibodies are encoded by immunoglobulin genes that have been genetically engineered so that the light and heavy chain genes are composed of immunoglobulin gene segments belonging to different species. For example, the variable (V) segments of the genes from a mouse monoclonal antibody may be joined to human constant (C) segments. Such a chimeric antibody is likely to be less antigenic to a human than antibodies with mouse constant regions as well as mouse variable regions.

As used herein, the term chimeric antibody also refers to an antibody that includes an immunoglobulin that has a human-like framework and in which any constant region present has at least about 85%-90%, and preferably about 95% polypeptide sequence identity to a human immunoglobulin constant region, a so-called "humanized" immunoglobulin (see, for example, PCT Publication WO 90/07861 ). Hence, all parts of such a "humanized" immunoglobulin, except possibly the complementarity determining regions (CDRs), are substantially identical to corresponding parts of one or more native human immunoglobulin sequences. Where necessary, framework residues may also be replaced with those within or across species especially if certain framework residues are found to affect the structure of the CDRs. A chimeric antibody may also contain truncated variable or constant regions.

The term "framework region," as used herein, refers to those portions of immunoglobulin light and heavy chain variable regions that are relatively conserved (*i.e.*, other than the CDRs) among different immunoglobulins in a single species, as defined by Kabat, et al., (1987); Sequences of Proteins of Immunologic Interest, 4th Ed., U.S. Dept. Health and Human Services). As used herein, a "human-like framework region" is a framework region that in each existing chain comprises at least about 70 or more amino acid residues, typically 75 to 85 or more residues, identical to those in a human immunoglobulin.

Human constant region DNA sequences can be isolated in accordance with well-known procedures from a variety of human cells, but preferably from immortalized B-cells. The variable regions or CDRs for producing the chimeric immunoglobulins of the present invention may be similarly derived from monoclonal antibodies capable of binding to the human type amyloid, and will be produced in any convenient mammalian system, including mice, rats, rabbits, human cell lines, or other vertebrates capable of producing antibodies by well-known methods. Variable regions or CDRs may be produced synthetically, by standard recombinant methods, including polymerase chain reaction ("PER") or through phage-display libraries. For phage display methods, see for example, McCafferty et al., Nature 348:552-554 (1990); Clackson et al., Nature 352:624-628 and Marks et al., Biotechnology 11:1145- 1149 (1993). Suitable prokaryotic systems such as bacteria, yeast and phage may be employed.

Suitable source cells for the DNA sequences and host cells for immunoglobulin expression and secretion can be obtained from a number of sources, such as the American Type Culture Collection ("Catalogue of Cell Lines and Hybridomas," Fifth edition (1985) Rockville, Maryland, U.S.A.).

In addition to the chimeric and "humanized" immunoglobulins specifically described herein, other substantially identical modified immunoglobulins can be readily designed and manufactured utilizing various recombinant DNA techniques well known to those skilled in the art. In general, modifications of the genes may be readily accomplished by a variety of well-known techniques, such as PCR and site-directed mutagenesis (see, Gillman and Smith, Gene 8:81-97 (1979) and S. Roberts et al., Nature 328:731-734 (1987)).

Alternatively, polypeptide fragments comprising only a portion of the primary immunoglobulin structure may be produced. For example, it may be desirable to produce immunoglobulin polypeptide fragments that possess one or more immunoglobulin activities in addition to, or other than, antigen recognition (*e.g.*, complement fixation).

Immunoglobulin genes, in whole or in part, may also be combined with functional regions from other genes (*e.g*., enzymes), or with other molecules such as toxins, labels and targeting moieties to produce fusion proteins (*e.g.*, "immunotoxins") having novel properties. In these cases of gene fusion, the two components are present within the same polypeptide chain. Alternatively, the immunoglobulin or fragment thereof may be chemically bonded to the toxin or label by any of a variety of well-known chemical procedures. For example, when the label or cytotoxic agent is a protein and the second component is an intact immunoglobulin, the linkage may be by way of heterobifunctional cross-linkers, *e.g.,* SPDP, carbodiimide, glutaraldehyde, or the like.

Suitable labels include, for example, radionuclides, enzymes, substrates, cofactors, inhibitors, fluorescers, chemiluminescers, magnetic particles. See, for examples of patents teaching the use of such labels, U.S. Patent Nos. 3,817,837; 3,850,752; 3,939,350; 3,996,345; 4,277,437; 4,275,149; and 4,366,241.

Immunotoxins, including single chain molecules, may also be produced by recombinant means. Production of various immunotoxins is well-known with the art, and methods can be found, for example in "Monoclonal Antibody-Toxin Conjugates: Aiming the Magic Bullet," Thorpe et al., Monoclonal Antibodies in Clinical Medicine, Academic Press, pp. 168-190 (1982); E. Vitetta, Science (1987) 238:1098-1104; and G. Winter and C. Milstein, Nature (1991) 349:293-299.

Additional techniques for preparing immunoglobulins and immunoglobulin fragments are described in V.S. Malik et al., Antibody Techniques (Academic Press, 1994); C.A.K. Borrebaeck, Antibody Engineering: Breakthroughs in Molecular Biology (Oxford Univ. Press, 1995); and P.J. Delves et al., Antibody Production: Essential Techniques (John Wiley & Sons, 1997).

"Opsonize", as used herein, refers to the binding of an immunoglobulin polypeptide to a particular target, particularly epitopes found on deposits of amyloid fibrils, such that the antibody and targets together are recognized as "foreign" by the host's cellular immune system. In other words the binding of the immunoglobulin of the present invention enhances the phagocytization of the amyloid fibrils.

"Amyloidosis", as used herein, is intended to refer to any condition that is characterized by the presence of amyloid material. Such material may be in the form of an amyloidoma or more disperse amyloid deposits or fibrils.

### Pharmaceutical Compositions

The pharmaceutical compositions for therapeutic treatment according to the present invention are intended for parenteral, oral or local administration. Preferably, the pharmaceutical compositions are administered parenterally, *e.g.,* intravenously, subcutaneously, intradermally, or intramuscularly. As the blood brain barrier is impermeable to IgG (see U. Bickel et al., 1994 Bioconjug. Chem. 5: 119-25), delivery of antibodies to overcome the blood-brain barrier (BBB) may be achieved through liposomal or micellar delivery of the antibody to the desired site. Alternatively, the agents of this invention can be delivered directly into the cerebrospinal fluid (see for example L.C. Walker et al., 1994 J. Neuropathol. Exp. Neurol. 53: 377-83). For other delivery mechanisms, refer to P.M. Friden, 1996 U.S. Patent No. 5,527,527 and W.M. Pardridge, 1991 U.S. Patent No. 5,004,697.

Thus, the invention provides compositions for parenteral administration which comprise a solution of the anti-human IgLC amyloid fibril immunoglobulin polypeptide dissolved or suspended in a pharmaceutically acceptable carrier, preferably an aqueous carrier. A variety of aqueous carriers may be used, *e.g.,* water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of anti-amyloid immunoglobulin polypeptides in the pharmaceutical formulations can vary widely, *i.e.,* from less than about 1%, usually at or at least about 10-15% to as much as 50% or more by weight, and will be selected primarily by fluid volumes, viscosities, etc., in an accordance with the particular mode of administration selected.

Without undue experimentation, one of ordinary skill in the art could determine the quantity of immunoglobulin polypeptides that would be effective in adequately opsonizing an amyloidoma. Amounts effective for this use will depend on, *e.g.,* the nature of the anti-amyloid immunoglobulin polypeptide composition, the manner of administration, the stage and severity of the disease being treated, the weight and general state of health of the patient, and the judgment of the prescribing physician. A typical single dose of 0.5 mg/kg could generally be used. It must be kept in an mind that the anti-amyloid immunoglobulin polypeptide and peptide compositions derived therefrom may be employed in serious disease states, that is, life-threatening or potentially life-threatening situations. In such cases it is possible and may be felt desirable by the treating physician to administer substantial excesses of these compositions.

Treatment of humans with amyloidosis could also be applied to animals susceptible to amyloidosis, such as cows or chickens. Thus, references to human patients herein apply also to non-human patients.

The immunoglobulin polypeptides, as defined herein, are anti-amyloid mAbs directed toward an amyloidoma or components or precursors thereof. The mAbs can be raised against IgLC variable region domains or, preferably, against the IgLC subsets κ1, κ4, λ8, or combinations thereof. The administration to humans of immunoglobulin polypeptides that are substantially non-human may elicit anti-antibody responses. Thus, it may be desirable to prepare anti-IgLC immunoglobulin polypeptides which are substantially human. By "substantially human" is meant an antibody or binding fragment thereof comprised of amino acid sequences which are at least about 50% human in origin, at least 70 to 80% more preferred, and about 95-99% or more human most preferred, particularly for repeated administrations over a prolonged period as may be necessary to treat established cases of amyloidosis. As used herein, human antibody is meant to include antibodies of entirely human origin as well as those which are substantially human, unless the context indicates otherwise.

Monoclonal antibodies can also be raised against synthetic amyloid fibrils. Recombinant light chain, variable region peptides are isolated and purified *in vitro* using standard techniques. Synthetic fibrils are then prepared from the peptides using techniques such as those described by Wall et al., "In vitro Immunoglobulin Light Chain Fibrillogenesis," METHODS IN ENZYMOLOGY, Vol. 309 (In Press). Antibodies are then raised against the synthetic fibrils using standard immunization techniques, typically in mice or rabbits. Monoclonal cell lines secreting anti-fibril antibodies are produced using standard hybridoma techniques.

The anti-amyloid immunoglobulin polypeptides may be prepared by any of a number of well-known techniques. For instance, they may be prepared by immunizing an animal with purified or partially purified human amyloid. The animals immunized can be any one of a variety of species which are capable of immunologically recognizing epitopes characteristic of the human type amyloid extracellular domain, such as murine, lagomorph, equine, etc.

Monoclonal antibodies may be prepared by immortalizing cells comprising nucleic acid sequences which encode immunoglobulin polypeptides or portions thereof that bind specifically to antigenic determinants characteristic of the extracellular domain of the human type amyloid. The immortalization process can be carried out by hybridoma fusion techniques, by viral transformation of antibody-producing lymphocytes, recombinant DNA techniques, or by techniques that combine cell fusion, viral transformation and/or recombinant DNA methodologies. Immunogens to raise the monoclonal antibodies include synthetic amyloid fibrils as described, for example by, A. Lomakin et al., 1997 Proc. Nat'l Acad. Sci. USA 94: 7942-7.

As the generation of human anti-amyloid monoclonal antibodies may be difficult with conventional immortalization techniques, it may be desirable to first make non-human antibodies and then transfer via recombinant DNA techniques the antigen binding regions of the non-human antibodies, *e.g*., the Fab, complementarity determining regions (CDRs) or hypervariable regions, to human constant regions (Fc) or framework regions as appropriate to produce substantially human molecules. Such methods are generally known in the art and are described in, for example, U.S. 4,816,397, PCT publication WO 90/07861, and EP publications 173494 and 239400. . However, completely human antibodies can be produced in transgenic animals. The desired human immunoglobulin genes or gene segments can be isolated, for example by PCR from human B cells, the DNA cloned into appropriate vectors for expression in eukaryotic cells and the cloned DNA introduced into animals to produce transgenics. Animals suitable for the production of transgenics expressing human immunoglobulin include mice, rats, rabbits and pigs with rodents of transgenics that express human immunoglobulins should preferably have one or more of their endogenous immunoglobulin loci inactivated or "knocked-out" to facilitate identification and isolation of the human antibodies (See *e.g.,* Lonberg, et al. Nature 368:856-859 (1994)).

The resulting chimeric antibodies or chimeric immunoglobulin polypeptides that bind to human amyloid are also within the scope of the present invention. A typical therapeutic chimeric antibody would be a hybrid protein consisting of the variable (V) or antigen-binding domain from a mouse immunoglobulin specific for a human amyloid antigenic determinant, and the constant (C) or effector domain from a human immunoglobulin, although domains from other mammalian species may be used for both variable and constant domains. As used herein, the therm "chimeric antibody" also refers to antibodies coded for by immunoglobulin genes in which only the CDRs are transferred from the immunoglobulin that specifically recognizes the antigenic determinants, the remainder of the immunoglobulin gene being derived from a human (or other mammalian, as desired) immunoglobulin gene. As discussed before, this type of chimeric antibody is referred to as a "humanized" (in the case of a human immunoglobulin gene being used) antibody. Also considered are recombinant human antibodies that do not contain sequences of another species.

The hypervariable regions of the variable domains of the anti-amyloid immunoglobulin polypeptides comprise a related aspect of the invention. The hypervariable regions, or CDRs, in conjunction with the framework regions (those portions of immunoglobulin light and heavy chain variable regions that are relatively conserved among different immunoglobulins in a single species), enable the anti-amyloid immunoglobulin polypeptides to recognize and thus bind to human amyloid. The hypervariable regions can be cloned and sequenced. Once identified, these regions that confer specific recognition of human amyloid can then be cloned into a vector for expression in a host as part of another immunoglobulin molecule or as a fusion protein, *e.g.,* a carrier molecule which functions to enhance immunogenicity of the cloned idiotype.

The anti-amyloid immunoglobulin polypeptides will generally be used intact, or as immunogenic fragments, such Fv, Fab, F(ab')₂ fragments. The fragments may be obtained from antibodies by conventional techniques, such as by proteolytic digestion of the antibody using, *e.g.,* pepsin, papain or other proteolytic enzymes, or by recombinant DNA techniques in which a gene or portion thereof encoding the desired fragment is cloned or synthesized, and expressed in a variety of hosts.

Those skilled in the art will realize that "anti-idiotypic" antibodies can be produced by using a specific immunoglobulin as an immunogen in accordance with standard techniques. For example, infection or immunization with an amyloid fibril or fragment thereof, induces a neutralizing immunoglobulin, which has on its Fab variable region combining site an image of the amyloid that is unique to that particular immunoglobulin, *i.e.,* an idiotype. Immunization with such an anti-amyloid immunoglobulin induces an anti-idiotype antibody, which has a conformation at its combining site that mimics the structure of the original amyloid antigen. These anti-idiotype antibodies may therefore be used instead of the amyloid antigen. See, for example, Nisonoff (1991) J. Immunol. 147:2429-2438.

The following working examples specifically point out preferred embodiments of the present invention, and are not to be construed as limiting in any way the remainder of the disclosure. Other generic configurations will be apparent to one skilled in the art.

### Example 1 Unassisted Resolution of Human IgLC Amyloid in Murine Host

Human IgLC amyloid was extracted and purified from infected organs obtained during an autopsy. The first experiments involved transplanting 50-200 mg of this amyloid material into a Balb/c mouse. The amyloid mass, or "amyloidoma," was prepared in sterile PBS by serial sonication and grinding steps in order to produce a fine suspension of amyloid fibrils complete with the accessory molecules found *in vivo.* This procedure was performed to allow the amyloid to be injected into the mice through a wide-gauge hypodermic needle.

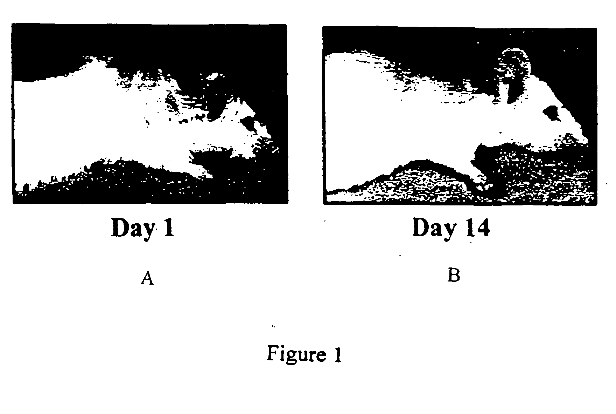

The amyloid material, equivalent to 10% of the body weight of the animal, was injected into mice (under anesthetic) between the scapula, which resulted in a large mass being visible (see Figure 1A). The mouse required 15-18 days to achieve the complete removal of the amyloidoma (see Figure 1B), after which the animal appeared healthy and lived a normal life span. The removal of the amyloidoma was determined subjectively by the experimenter; by simply palpating the injection site, an amyloidoma, like a hard pea, can be easily felt under the skin.

### Example 2 Involvement of Both Antibody-Mediated and Cellular Immunity in the Removal of Amyloidomas

The involvement of anti-amyloid antibodies in the removal of amyloidomas was shown by screening serum from a mouse previously injected with amyloid material against a sample of the injected material. This was done by Western blot analysis using suitable dilutions of the mouse serum as the primary antibody. It was shown that there were antibodies to every component of the amyloid matrix, *i.e*., every band on the gel was stained by the mouse serum, even at a 10,000-fold serum dilution (data not shown).

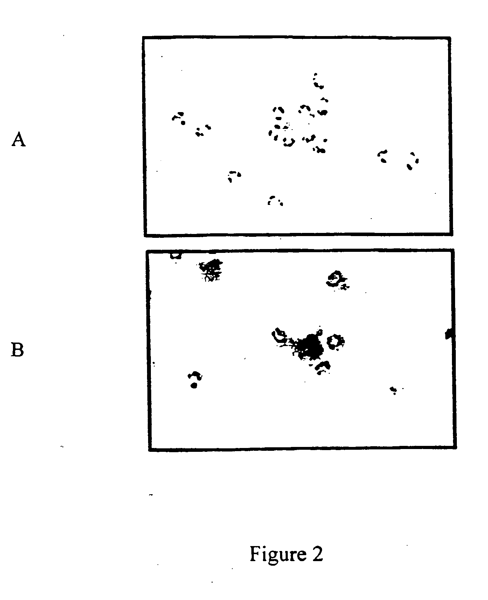

The involvement of a cellular component was demonstrated by *in vitro* neutrophil binding assays (see Figures 2A and 2B) and by using knockout-mutant mouse strains (data not shown). Figures 2A and 2B show human neutrophils adhering to human amyloid after the amyloid was treated with mouse anti-human IgLC mAbs. This shows that the mouse mAb can bind to human amyloid as well as attract human neutrophils.

Studies of knockout-mutant mouse strains further support a finding of antibody involvement in amyloid removal. First, *scid*/*scid* mice, which lack B and T lymphocytes, were unable to remove an injected amyloidoma even after three months (data not shown). Second, CD18 knockout animals were unable to remove the amyloidoma as rapidly as normal animals. CD18 knockout animals are 97% deficient in CD 18, a cell surface integrin found on granulocyte/macrophage lineages. Although these cells cannot leave the circulation, the animals are B and T cell competent and can therefore mount an antibody response. Third, nude mice, which have no white blood cells, were unable to remove the amyloidoma.

Furthermore, amyloid that had been incubated with amyloid-reactive serum from another mouse, when implanted into the second mouse, was removed within 4 days. In this experiment a Balb/c mouse was injected with 50 mg HIG amyloid and left for 1 week, after which it was bled by tail-vein clipping. The blood was spun down at 1500 rpm and the cells removed by aspiration. The plasma was stored at 4 °C until used. Another preparation of HIG amyloid (100 mg) was prepared by suspending in sterile PBS to which was added 1 ml of plasma from the previous mouse. This preparation was then injected into a second mouse (Balb/c) and the amyloid was removed in 4 days. Thus, it was concluded that the process could be sped up by opsonizing the material prior to injection.

### Example 3 ELISA Screening of IgLC Subsets

A systematic study was performed using ELISA techniques to screen a large number of human extracted amyloid samples using mAbs raised against the IgLC subsets (λ1, λ2, λ3, λ4, λ5, λ6, κ1, κ2, κ3, κ4, free κ and λ and total κ and λ). Interestingly, it was found that more often than not, the amyloids tested positive with mAbs specific for their own subtype, the total κ or λ antibodies and a κ1(57-18H12), κ4(11-1F4) and λ8(31-8C7) mAb. These latter three reagents were found to react in a non-subgroup specific manner, *i.e.,* κ1 reacted with amyloids comprised of IgLCs other than κ1; and the other two mAbs exhibit the same quality. This shows that the epitope recognized by these antibodies may be a general feature of amyloid fibrils, indicating the possibility of a shared amyloid epitope that can be targeted.

### Example 4 Immunochemical Staining

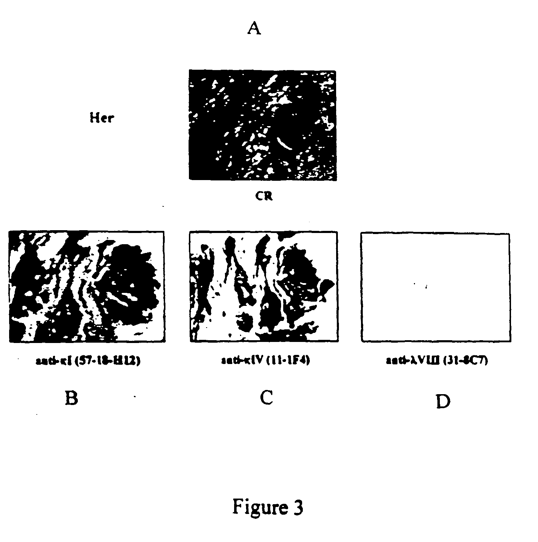

Tissue samples from amyloid patients were stained using standard immunochemical techniques and a similar binding phenomenon was observed. Figures 3A-3D show that anti-κ1 binds to the κ1 amyloid and, surprisingly, that the anti-κ4 reacts with the κ1 amyloid, suggesting an amyloid epitope that these antibodies may recognize. Additionally, the anti-κ4 reacts with λ-containing amyloid (not illustrated). This is an example of cross-isotype reactivity. However, the results from the ELISA and the immunohistochemistry were not always consistent. This is likely due to the inherent difference in what you are looking at, *i.e*., ELISA is a liquid phase binding assay using extracted purified amyloid, whereas immunohistochemistry is performed on fixed tissue sections on a slide.

### Example 5 In Vivo Studies of Anti-IgLC Subgroups

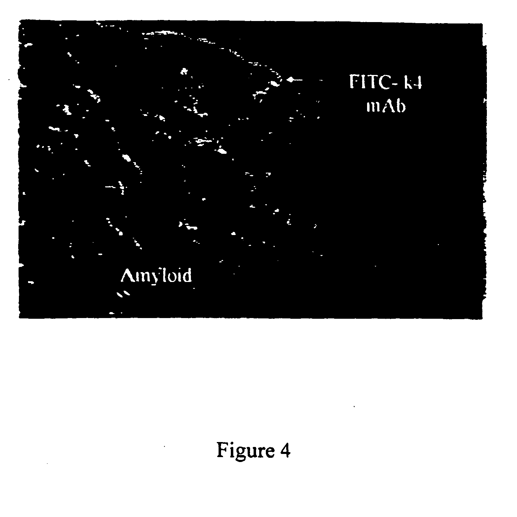

0.1 mg of one of three antibodies - κ1, κ4, or λ8, identified above - was injected into the thigh of a mouse into which amyloid had been introduced in the form of an amyloidoma as described above. The κ1 and κ4 reagents resulted in the complete removal by the host of most amyloid fibril species tested within 7 days (as little as 4 days for certain sources of amyloid). Figure 4 shows fluoresceinated κ4 mAb binding to human amyloid.

The λ8 reagent, which is reactive in certain instances in both *in vitro* studies (above), increased the resolution of amyloidomas by up to about 10% in *in vivo* experiments.

### Example 6 In Vivo Studies of Anti-IgLC Subgroups

Human amyloid was isolated from a patient with inflammation-associated, AA-amyloid and prepared for injection into Balb/C mice by repeated sonication and grinding in order to permit its injection into the mouse (see Example 1). Immediately after the injection of 100 mg of human AA-amyloid extract, the mice were treated with 100 µg of κ4 mAb, anti-AA mAb, no mAb and non-specific control mAb (anti-free κ). Complete resolution of the material was observed with 48 hours in the animals that had been treated with the κ4 and anti-AA mAbs. In contrast, the control animals had a large mass of amyloid remaining at the site of injection.

### Example 7 Production of Specific Anti-Amvloid Fibril mAbs

Synthetic amyloid fibrils were prepared *in vitro* and used as an immunogen in mice to produce a first generation of anti-amyloid fibril mAbs. Briefly, recombinant λ6-light chain, variable region peptides were produced, isolated and purified using a bacterial expression system and standard protein purification techniques. Synthetic fibrils were prepared from these peptides by extended periods of agitation in solution as described, for example, in Wall et al., "In vitro Immunoglobulin Light Chain Fibrillogenesis," METHODS IN ENZYMOLOGY, Vol. 309 (In Press) . Fibrils were concentrated by centrifugation at 17,000 × g for 20 minutes at room temperature.

The concentrated fibrils were then used to immunize Balb/c mice over a period of several weeks. Monoclonal cell lines secreting anti-fibril antibodies were produced using standard hybridoma techniques. The resultant antibodies have demonstrable anti-fibril activity based upon ELISA assays, described in Example 3. These antibodies reacted with 99% of all human IgLC amyloid extracts tested to date irrespective of the nature of the isotype or subgroup of the precursor protein when tested by ELISA. Similarly, the antibodies reacted in an ELISA format with isolated murine AA-amyloid and synthetic fibrils composed of a peptide derived from the Alzheimer's protein Aβ [Aβ(25-35)].

It should be understood that the foregoing discussion and examples merely present a detailed description of certain preferred embodiments.

## Claims

1. The use of:
a) a therapeutically effective dose of at least one antibody or immunoglobulin polypeptide or a fragment thereof which binds to human IgLC amyloid fibril; and
b) a pharmaceutically acceptable carrier;
for the manufacture of a medicament for the treatment of an amyloid deposition disease in a human patient.

2. The use of claim 1, wherein the antibody or immunoglobulin polypeptide or fragment thereof is raised against an immunoglobulin light-chain.

3. The use of claim 1 or 2, wherein the antibody or immunoglobulin polypeptide or fragment thereof is a monoclonal antibody.

4. The use of claim 3, wherein the monoclonal antibody is a completely human antibody.

5. The use of claim 3, wherein the monoclonal antibody is a humanized antibody.

6. The use of claim 3, wherein the monoclonal antibody is a chimeric antibody.

7. The use of claim 6, wherein the chimeric antibody is a humanized antibody.

8. The use of claim 3, wherein the antibody is a labeled antibody.

9. The use of claim 1, wherein the antibody or immunoglobulin fragment is a Fv fragment, Fab fragment, F(ab") fragment, F(ab')₂ fragment, or SvFv fragment.

10. The use of claim 1, wherein the antibody or immunoglobulin polypeptide or fragment is a single chain antibody.

11. The use of claim 1, wherein the antibody or immunoglobulin polypeptide or fragment has cross-isotype reactivity.

12. The use of claim 1, wherein the antibody or immunoglobulin polypeptide or fragment thereof has been raised against synthetic amyloid fibrils.

13. A composition comprising a therapeutically effective dose of at least one antibody or immunoglobulin polypeptide or a fragment thereof which binds to human IgLC fibril; and
b) a pharmaceutically acceptable carrier;
for use in the treatment of an amyloid deposition disease in a human patient.

14. The composition of claim 13, wherein the antibody or immunoglobulin polypeptide or fragment thereof is raised against an immunoglobulin light-chain.

15. The composition of claim 13 or 14, wherein the antibody or immunoglobulin polypeptide or fragment thereof is a monoclonal antibody.

16. The composition of claim 15, wherein the monoclonal antibody is a completely human antibody.

17. The composition of claim 15, wherein the monoclonal antibody is a humanized antibody.

18. The composition of claim 15, wherein the monoclonal antibody is a chimeric antibody.

19. The composition of claim 18, wherein the chimeric antibody is a humanized antibody.

20. The composition of claim 15, wherein the antibody is a labeled antibody.

21. The composition of claim 13, wherein the antibody or immunoglobulin fragment is a Fv fragment, Fab fragment, F(ab") fragment, F(ab')₂ fragment, or SvFv fragment.

22. The composition of claim 13, wherein the antibody or immunoglobulin polypeptide or fragment is a single chain antibody.

23. The composition of claim 13, wherein the antibody or immunoglobulin polypeptide or fragment has cross-isotype reactivity.

24. The composition of claim 13, wherein the antibody or immunoglobulin polypeptide or fragment thereof has been raised against synthetic amyloid fibrils.

25. A pharmaceutical composition for use in therapy comprising a therapeutically effective dose of at least one antibody or immunoglobulin polypeptide or a fragment thereof, wherein the antibody or immunoglobulin polypeptide or fragment thereof binds to human IgLC fibril and is effective to enhance the cellular immune response of a patient to remove disease associated amyloid fibril deposits.

26. The pharmaceutical composition of claim 25, wherein the antibody or immunoglobulin polypeptide or fragment thereof is a monoclonal antibody or fragment thereof.

27. The pharmaceutical composition of claim 26, wherein the monoclonal antibody is a completely human antibody.

28. The pharmaceutical composition of claim 26, wherein the monoclonal antibody is a humanized antibody.

29. The pharmaceutical composition of claim 26, wherein the antibody is a labeled antibody.

30. The pharmaceutical composition of claim 25, wherein the antibody or immunoglobulin fragment is a Fv fragment, Fab fragment, F(ab") fragment, F(ab')₂ fragment, or SvFv fragment.

31. The pharmaceutical composition of claim 25, wherein the antibody or immunoglobulin polypeptide or fragment thereof is a single chain antibody.

32. The pharmaceutical composition of claim 25, wherein the antibody or immunoglobulin polypeptide or fragment thereof has cross-isotype reactivity.

33. The pharmaceutical composition of any one of claims 25 to 32, wherein the composition further comprises a carrier.

## Patentansprüche

1. Verwendung
a) einer therapeutisch wirksamen Dosis mindestens eines Antikörper- oder Immunglobulin-Polypeptids oder Fragments davon, das an humane IgLC-Amyloidfibrillen bindet; und
b) einer pharmazeutisch zulässigen Trägersubstanz;
für die Herstellung eines Arzneimittels zur Behandlung einer Erkrankung mit Amyloidablagerung bei einem menschlichen Patienten.

2. Verwendung nach Anspruch 1, wobei das Antikörper- oder Immunglobulin-Polypeptid oder Fragment davon gegen eine Immunglobulin-Leichtkette gerichtet ist.

3. Verwendung nach Anspruch 1 oder 2, wobei das Antikörper- oder Immunglobulin-Polypeptid oder Fragment davon ein monoklonaler Antikörper ist.

4. Verwendung nach Anspruch 3, wobei der monoklonale Antikörper ein vollständig humaner Antikörper ist.

5. Verwendung nach Anspruch 3, wobei der monoklonale Antikörper ein humanisierter Antikörper ist.

6. Verwendung nach Anspruch 3, wobei der monoklonale Antikörper ein chimärer Antikörper ist.

7. Verwendung nach Anspruch 6, wobei der chimäre Antikörper ein humanisierter Antikörper ist.

8. Verwendung nach Anspruch 3, wobei der Antikörper ein gelabelter Antikörper ist.

9. Verwendung nach Anspruch 1, wobei das Antikörper- oder Immunglobulin-Fragment ein Fv-Fragment, Fab-Fragment, F(ab")-Fragment, F(ab')₂-Fragment oder SvFv-Fragment ist.

10. Verwendung nach Anspruch 1, wobei das Antikörper- oder Immunglobulin-Polypeptid oder Fragment davon ein einkettiger Antikörper ist.

11. Verwendung nach Anspruch 1, wobei das Antikörper- oder Immunglobulin-Polypeptid oder Fragment davon Isotypen-Kreuzreaktivität aufweist.

12. Verwendung nach Anspruch 1, wobei das Antikörper- oder Immunglobulin-Polypeptid oder Fragment davon gegen synthetische Amyloidfibrillen gerichtet ist.

13. Zusammensetzung, beinhaltend eine therapeutisch wirksame Dosis mindestens eines Antikörper- oder Immunglobulin-Polypeptids oder Fragments davon, das an humane IgLC-Amyloidfibrillen bindet; und
b) eine pharmazeutisch zulässige Trägersubstanz; zur Verwendung bei der Behandlung einer Erkrankung mit Amyloidablagerung bei einem menschlichen Patienten.

14. Zusammensetzung nach Anspruch 13, wobei das Antikörper- oder Immunglobulin-Polypeptid oder Fragment davon gegen eine Immunglobulin-Leichtkette gerichtet ist.

15. Zusammensetzung nach Anspruch 13 oder 14, wobei das Antikörper- oder Immunglobulin-Polypeptid oder Fragment davon ein monoklonaler Antikörper ist.

16. Zusammensetzung nach Anspruch 15, wobei der monoklonale Antikörper ein vollständig humaner Antikörper ist.

17. Zusammensetzung nach Anspruch 15, wobei der monoklonale Antikörper ein humanisierter Antikörper ist.

18. Zusammensetzung nach Anspruch 15, wobei der monoklonale Antikörper ein chimärer Antikörper ist.

19. Zusammensetzung nach Anspruch 18, wobei der chimäre Antikörper ein humanisierter Antikörper ist.

20. Zusammensetzung nach Anspruch 15, wobei der Antikörper ein gelabelter Antikörper ist.

21. Zusammensetzung nach Anspruch 13, wobei das Antikörper- oder Immunglobulin-Fragment ein Fv-Fragment, Fab-Fragment, F(ab")-Fragment, F(ab')₂-Fragment oder SvFv-Fragment ist.

22. Zusammensetzung nach Anspruch 13, wobei das Antikörper- oder Immunglobulin-Polypeptid oder Fragment davon ein einkettiger Antikörper ist.

23. Zusammensetzung nach Anspruch 13, wobei das Antikörper- oder Immunglobulin-Polypeptid oder Fragment davon Isotypen-Kreuzreaktivität aufweist.

24. Zusammensetzung nach Anspruch 13, wobei das Antikörper- oder Immunglobulin-Polypeptid oder Fragment davon gegen synthetische Amyloidfibrillen gerichtet ist.

25. Pharmazeutische Zusammensetzung zur Verwendung in der Therapie, beinhaltend eine therapeutisch wirksame Dosis mindestens eines Antikörper- oder Immunglobulin-Polypeptids oder Fragments davon, wobei das Antikörper- oder Immunglobulin-Polypeptid oder Fragment davon an humane IgLC-Fibrillen bindet und dafür wirksam ist, die zelluläre Immunantwort eines Patienten zu verbessern, um krankheitsbedingte Amyloidfibrillen-Ablagerungen zu beseitigen.

26. Pharmazeutische Zusammensetzung nach Anspruch 25, wobei das Antikörper- oder Immunglobulin-Polypeptid oder Fragment davon ein monoklonaler Antikörper oder Fragment davon ist.

27. Pharmazeutische Zusammensetzung nach Anspruch 26, wobei der monoklonale Antikörper ein vollständig humaner Antikörper ist.

28. Pharmazeutische Zusammensetzung nach Anspruch 26, wobei der monoklonale Antikörper ein humanisierter Antikörper ist.

29. Pharmazeutische Zusammensetzung nach Anspruch 26, wobei der Antikörper ein gelabelter Antikörper ist.

30. Pharmazeutische Zusammensetzung nach Anspruch 25, wobei das Antikörper- oder Immunglobulin-Fragment ein Fv-Fragment, Fab-Fragment, F(ab")-Fragment, F(ab')₂-Fragment oder SvFv-Fragment ist.

31. Pharmazeutische Zusammensetzung nach Anspruch 25, wobei das Antikörper- oder Immunglobulin-Polypeptid oder Fragment davon ein einkettiger Antikörper ist.

32. Pharmazeutische Zusammensetzung nach Anspruch 25, wobei das Antikörper- oder Immunglobulin-Polypeptid oder Fragment davon Isotypen-Kreuzreaktivität aufweist.

33. Pharmazeutische Zusammensetzung nach einem der Ansprüche 25 bis 32, wobei de Zusammensetzung ferner eine Trägersubstanz beinhaltet.

## Revendications

1. L'utilisation de :
a) une dose thérapeutiquement efficace d'au moins un anticorps ou polypeptide d'immunoglobuline ou d'un fragment de ceux-ci qui se lie à une fibrille amyloïde IgLC humaine, et
b) un vecteur pharmaceutiquement acceptable,
pour la fabrication d'un médicament destiné au traitement d'une maladie de dépôt d'amyloïdes chez un patient humain.

2. L'utilisation selon la Revendication 1, où l'anticorps ou le polypeptide d'immunoglobuline ou un fragment de ceux-ci est dirigé contre une chaîne légère d'immunoglobine.

3. L'utilisation selon la Revendication 1 ou 2, où l'anticorps ou le polypeptide d'immunoglobuline ou un fragment de ceux-ci est un anticorps monoclonal.

4. L'utilisation selon la Revendication 3, où l'anticorps monoclonal est un anticorps entièrement humain.

5. L'utilisation selon la Revendication 3, où l'anticorps monoclonal est un anticorps humanisé.

6. L'utilisation selon la Revendication 3, où l'anticorps monoclonal est un anticorps chimérique.

7. L'utilisation selon la Revendication 6, où l'anticorps chimérique est un anticorps humanisé.

8. L'utilisation selon la Revendication 3, où l'anticorps est un anticorps marqué.

9. L'utilisation selon la Revendication 1, où l'anticorps ou le polypeptide d'immunoglobuline est un fragment Fv, un fragment Fab, un fragment F(ab"), un fragment F(ab')₂ ou un fragment SvFv.

10. L'utilisation selon la Revendication 1, où l'anticorps ou le polypeptide d'immunoglobuline ou le fragment est un anticorps à chaîne unique.

11. L'utilisation selon la Revendication 1, où l'anticorps ou le polypeptide d'immunoglobuline ou le fragment présente une réactivité entre isotypes.

12. L'utilisation selon la Revendication 1, où l'anticorps ou le polypeptide d'immunoglobuline ou un fragment de ceux-ci a été dirigé contre des fibrilles amyloïdes synthétiques.

13. Une composition contenant une dose thérapeutiquement efficace d'au moins un anticorps ou polypeptide d'immunoglobuline ou d'un fragment de ceux-ci qui se lie à une fibrille IgLC humaine, et
b) un vecteur pharmaceutiquement acceptable,
pour une utilisation dans le traitement d'une maladie de dépôt d'amyloïdes chez un patient humain.

14. La composition selon la Revendication 13, où l'anticorps ou le polypeptide d'immunoglobuline ou un fragment de ceux-ci est dirigé contre une chaîne légère d'immunoglobine.

15. La composition selon la Revendication 13 ou 14, où l'anticorps ou le polypeptide d'immunoglobuline ou un fragment de ceux-ci est un anticorps monoclonal.

16. La composition selon la Revendication 15, où l'anticorps monoclonal est un anticorps entièrement humain.

17. La composition selon la Revendication 15, où l'anticorps monoclonal est un anticorps humanisé.

18. La composition selon la Revendication 15, où l'anticorps monoclonal est un anticorps chimérique.

19. La composition selon la Revendication 18, où l'anticorps chimérique est un anticorps humanisé.

20. La composition selon la Revendication 15, où l'anticorps est un anticorps marqué.

21. La composition selon la Revendication 13, où l'anticorps ou le polypeptide d'immunoglobuline est un fragment Fv, un fragment Fab, un fragment F(ab"), un fragment F(ab')₂ ou un fragment SvFv.

22. La composition selon la Revendication 13, où l'anticorps ou le polypeptide d'immunoglobuline ou le fragment est un anticorps à chaîne unique.

23. La composition selon la Revendication 13, où l'anticorps ou le polypeptide d'immunoglobuline ou le fragment présente une réactivité entre isotypes.

24. La composition selon la Revendication 13, où l'anticorps ou le polypeptide d'immunoglobuline ou un fragment de ceux-ci a été dirigé contre des fibrilles amyloïdes synthétiques.

25. Une composition pharmaceutique à usage thérapeutique contenant une dose thérapeutiquement efficace d'au moins un anticorps ou polypeptide d'immunoglobuline ou d'un fragment de ceux-ci, où l'anticorps ou polypeptide d'immunoglobuline ou le fragment de ceux-ci se lie à une fibrille IgLC humaine et est efficace pour améliorer la réponse immunitaire cellulaire d'un patient au retrait de dépôts de fibrilles amyloïdes associés à une maladie.

26. La composition pharmaceutique selon la Revendication 25, où l'anticorps ou le polypeptide d'immunoglobuline ou un fragment de ceux-ci est un anticorps monoclonal ou un fragment de celui-ci.

27. La composition pharmaceutique selon la Revendication 26, où l'anticorps monoclonal est un anticorps entièrement humain.

28. La composition pharmaceutique selon la Revendication 26, où l'anticorps monoclonal est un anticorps humanisé.

29. La composition pharmaceutique selon la Revendication 26, où l'anticorps monoclonal est un anticorps marqué.

30. La composition pharmaceutique selon la Revendication 25, où l'anticorps ou le fragment immunoglobuline est un fragment Fv, un fragment Fab, un fragment F(ab"), un fragment F(ab')₂ ou un fragment SvFv.

31. La composition pharmaceutique selon la Revendication 25, où l'anticorps ou le polypeptide d'immunoglobuline ou le fragment est un anticorps à chaîne unique.

32. La composition pharmaceutique selon la Revendication 25, où l'anticorps ou le polypeptide d'immunoglobuline ou un fragment de ceux-ci présente une réactivité entre isotypes.

33. La composition pharmaceutique selon l'une quelconque des Revendications 25 à 32, où la composition comprend en outre un vecteur.
